# EUROPEAN PATENT SPECIFICATION

(11) **EP 1 382 685 B1**
(45) Date of publication and mention of the grant of the patent: **02.07.2008**
(21) Application number: 03015148.4
(22) Date of filing: 04.07.2003
(51) Int. Cl.: C12P 13/08

(54) **Process for the fermentative preparation of L-amino acids using strains of the enterobacteriaceae family with overexpressed rseB gene**
Verfahren zur fermentativen Herstellung von L-Aminosäuren durch Enterobakteriaceae-Stämmen mit verstärkter Exprimierung des rseB-Gens
Procédé de production d'acides L-aminés par fermentation de souches de la famille Enterobacteriaceae surexprimant le gène rseB

(30) Priority: 10.07.2002 DE 10231115
(43) Date of publication of application: 21.01.2004
(73) Proprietor: Evonik Degussa GmbH, 40474 Düsseldorf (DE)
(72) Inventor: Rieping, Mechthild, Dr., 33619 Bielefeld (DE); Siebelt, Nicole, 33397 Rietberg (DE)

(56) References cited:
- EP-A- 0 643 135
- EP-A- 0 994 190
- WO-A-99/53035
- WO-A-03/008600
- WO-A-03/008612
- US-A- 4 278 765
- MISSIAKAS D ET AL.: "Modulation of the Escherichia coli sigmaE (RpoE) heat-shock transcription-factor activity by the RseA, RseB and RseC proteins." MOLECULAR MICROBIOLOGY, vol. 24, no. 2, April 1997 (1997-04), pages 355-371, XP008020247 ISSN: 0950-382X
- DE LAS PEÑAS A ET AL.: "The sigmaE-mediated response to extracytoplasmic stress in Escherichia coli is transduced by RseA and RseB, two negative regulators of sigmaE." MOLECULAR MICROBIOLOGY, vol. 24, no. 2, April 1997 (1997-04), pages 373-385, XP008017874 ISSN: 0950-382X
- COLLINET B ET AL.: "RseB binding to the periplasmic domain of RseA modulates the RseA:sigmaE interaction in the cytoplasm and the availability of sigmaE.RNA polymerase." THE JOURNAL OF BIOLOGICAL CHEMISTRY, vol. 275, no. 43, 27 October 2000 (2000-10-27), pages 33898-33904, XP002267869 ISSN: 0021-9258
- NITTA T ET AL.: "Function of the sigma(E) regulon in dead-cell lysis in stationary-phase Escherichia coli." JOURNAL OF BACTERIOLOGY, vol. 182, no. 18, September 2000 (2000-09), pages 5231-5237, XP002243444 ISSN: 0021-9193
- MICHAL G: "Biochemical pathways: an atlas of biochemistry and molecular biology" 1999 , JOHN WILEY & SONS INC. AND SPEKTRUM AKADEMISCHER VERLAG , NEW YORK - HEIDELBERG XP002242199 ISBN: 0-471-33130-9 * figure 3.8-2 * * figures 4.2-1, 4.5-1 and 4.5-2 *
- TAO H ET AL.: "Functional genomics: Expression analysis of Escherichia coli growing on minimal and rich media" JOURNAL OF BACTERIOLOGY, vol. 181, no. 20, October 1999 (1999-10), pages 6425-6440, XP002200195 ISSN: 0021-9193
- KRAEMER R: "Genetic and physiological approaches for the production of amino acids" JOURNAL OF BIOTECHNOLOGY, vol. 45, no. 1, 1996, pages 1-21, XP002178648 ISSN: 0168-1656
- JETTEN M S M ET AL.: "Recent advances in the physiology and genetics of amino acid-producing bacteria." CRC CRITICAL REVIEWS IN BIOTECHNOLOGY, vol. 15, no. 1, 1995, pages 73-103, XP000613291 ISSN: 0738-8551
- DEBABOV V G: "The threonine story" ADVANCES IN BIOCHEMICAL ENGINEERING, vol. 79, 2003, pages 113-136, XP008014933 ISSN: 0724-6145
- IKEDA M: "Amino acid production processes" ADVANCES IN BIOCHEMICAL ENGINEERING, vol. 79, 2003, pages 1-35, XP008014932 ISSN: 0724-6145
- HERMANN T ET AL.: "Improved L-threonine production with Escherichia coli" ECB11 (11th European congress on Biotechnology) Abstracts, 24-29 August 2003 (2003-08-24), Basel, CH. XP002267870
- LEE J-H ET AL.: "Global analyses of transcriptosomes and proteomes of a parent strain and an L-threonine-overproducing mutant strain" JOURNAL OF BACTERIOLOGY, vol. 185, no. 18, September 2003 (2003-09), pages 5442-5451, XP009022136 ISSN: 0021-9193
- IKEDA M ET AL.: "The Corynebacterium glutamicum genome: features and impacts on biotechnological processes." APPLIED MICROBIOLOGY AND BIOTECHNOLOGY. GERMANY AUG 2003, vol. 62, no. 2-3, August 2003 (2003-08), pages 99-109, XP001184751 ISSN: 0175-7598
- KALINOWSKI J ET AL.: "The complete Corynebacterium glutamicum ATCC 13032 genome sequence and its impact on the production of L-aspartate-derived amino acids and vitamins." JOURNAL OF BIOTECHNOLOGY, vol. 104, no. 1-3, 4 September 2003 (2003-09-04), pages 5-25, XP001184752 ISSN: 0168-1656
- PATTE J.-C.: 'Biosynthesis of threonine and lysine, in: Neidhardt et al. (editors) 'Escherichia coli and Salmonella typhimurium'', 1996, ASM PRESS, WASHINGTON DC, ISBN 1-55581-084-5 * pages 528-541 *

## Description

This invention relates to a process for the preparation of L-lysine and L-threonine, using strains of the Enterobacteriaceae family in which the rseB gene is overexpressed.

### Prior art

L-Amino acids, in particular L-threonine, are used in human medicine and in the pharmaceuticals industry, in the foodstuffs industry and very particularly in animal nutrition.

It is known that L-amino acids are prepared by fermentation of strains of Enterobacteriaceae, in particular Escherichia coli (E. coli) and Serratia marcescens. Because of their great importance, work is constantly being undertaken to improve the preparation processes. Improvements to the process can relate to fermentation measures, such as e.g. stirring and supply of oxygen, or the composition of the nutrient media, such as e.g. the sugar concentration during the fermentation, or the working up to the product form, by e.g. ion exchange chromatography, or the intrinsic output properties of the microorganism itself.

Methods of mutagenesis, selection and mutant selection are used to improve the output properties of these microorganisms. Strains which are resistant to antimetabolites, such as e.g. the threonine analogue α-amino-β-hydroxyvaleric acid (AHV), or are auxotrophic for metabolites of regulatory importance and produce L-amino acids, such as e.g. L-threonine, are obtained in this manner.

Methods of the recombinant DNA technique have also been employed for some years for improving the strain of strains of the Enterobacteriaceae family which produce L-amino acids, by amplifying individual amino acid biosynthesis genes and investigating the effect on the production. Summarizing information on the cell and molecular biology of Escherichia coli and Salmonella are to be found in Neidhardt (ed): Escherichia coli and Salmonella, Cellular and Molecular Biology, 2nd edition, ASM Press, Washington, D.C., USA (1995).

### Object of the invention

The inventors had the object of providing new measures for improved fermentative preparation of L-lysine and L-threonine.

### Description of the invention

The invention provides a process for the fermentative preparation of L-lysine and L-threonine, using microorganisms of the Enterobacteriaceae family which, in particular, already produce L-amino acids and in which the nucleotide sequence which codes for the rseB gene is overexpressed.

Where L-amino acids or amino acids are mentioned in the following, this means one or more amino acids, including their salts, chosen from the group consisting of L-threonine L-lysine. L-Threonine is particularly preferred.

The term "enhancement" in this connection describes the increase in the intracellular activity or concentration of one or more enzymes or proteins in a microorganism which are coded by the corresponding DNA, for example by increasing the number of copies of the gene or genes or alleles by at least one (1) copy, using a potent promoter or a gene or allele which codes for a corresponding enzyme or protein with a high activity, and optionally combining these measures.

By over-expression, the activity or concentration of the corresponding protein is in general increased by at least 10%, 25%, 50%, 75%, 100%, 150%, 200%, 300%, 400% or 500%, up to a maximum of 1000% or 2000%, based on that of the wild-type protein or the activity or concentration of the protein in the starting microorganism.

The invention provides a process for the preparation
a) of L-lysine or threonine, or feedstuffs additives comprising these compounds, by culture of microorganisms of the Enterobacteriaceae family in which the rseB gene or nucleotide sequences which code for it are over-expressed, in a medium under conditions suitable for the formation of the rseB gene product,
b) the desired L-amino acid can become concentrated in the medium or in the cells, and
c) the product containing the desired L-amino acid(s), is isolated, constituents of the fermentation broth and/or the biomass optionally remaining in the isolated product in an amount of ≥ 0 to 100%.

The microorganisms, in particular recombinant microorganisms, which the present invention provides can produce L-amino acids from glucose, sucrose, lactose, fructose, maltose, molasses, optionally starch, optionally cellulose or from glycerol and ethanol. They are representatives of the Enterobacteriaceae family chosen from the genera Escherichia, Erwinia, Providencia and Serratia. The genera Escherichia and Serratia are preferred. Of the genus Escherichia the species Escherichia coli and of the genus Serratia the species Serratia marcescens are to be mentioned in particular. Recombinant microorganisms are in general generated by transformation, transduction or conjugation with a vector which carries the desired gene.

Suitable strains, which produce L-threonine in particular, of the genus Escherichia, in particular of the species Escherichia coli, are, for example
- Escherichia coli H4578 (Applied Micobiology Biotechnology 29: 550-553 (1988))
- Escherichia coli KY10935 (Bioscience Biotechnology and Biochemistry 61(11): 1877-1882 (1997))
- Escherichia coli VNIIgenetika MG442 (US-A-4278,765)
- Escherichia coli VNIIgenetika M1 (US-A-4,321,325)
- Escherichia coli VNIIgenetika 472T23 (US-A-5,631,157)
- Escherichia coli BKIIM B-3996 (US-A-5,175,107)
- Escherichia coli kat 13 (WO 98/04715)
- Escherichia coli KCCM-10132 (WO 00/09660)

Suitable L-threonine-producing strains of the genus Serratia, in particular of the species Serratia marcescens, are, for example
- Serratia marcescens HNr21 (Applied and Environmental Microbiology 38(6): 1045-1051 (1979))
- Serratia marcescens TLr156 (Gene 57(2-3): 151-158 (1987))
- Serratia marcescens T2000 (Applied Biochemistry and Biotechnology 37(3): 255-265 (1992))

Strains from the Enterobacteriaceae family which produce L-threonine preferably have, inter alia, one or more genetic or phenotypic features chosen from the group consisting of: resistance to α-amino-β-hydroxyvaleric acid, resistance to thialysine, resistance to ethionine, resistance to α-methylserine, resistance to diaminosuccinic acid, resistance to α-aminobutyric acid, resistance to borrelidin or cyclopentane-carboxylic acid, resistance to rifampicin, resistance to valine analogues, such as, for example, valine hydroxamate, resistance to purine analogues, such as, for example, 6-dimethylaminopurine, a need for L-methionine, optionally a partial and compensatable need for L-isoleucine, a need for meso-diaminopimelic acid, auxotrophy in respect of threonine-containing dipeptides, resistance to L-threonine or threonine raffinate, resistance to L-homoserine, resistance to L-lysine, resistance to L-methionine, resistance to L-glutamic acid, resistance to L-aspartate, resistance to L-leucine, resistance to L-phenylalanine, resistance to L-serine, resistance to L-cysteine, resistance to L-valine, sensitivity to fluoropyruvate, defective threonine dehydrogenase, optionally an ability for sucrose utilization, enhancement of the threonine operon, enhancement of homoserine dehydrogenase I-aspartate kinase I, preferably of the feed back resistant form, enhancement of homoserine kinase, enhancement of threonine synthase, enhancement of aspartate kinase, optionally of the feed back resistant form, enhancement of aspartate semialdehyde dehydrogenase, enhancement of phosphoenol pyruvate carboxylase, optionally of the feed back resistant form, enhancement of phosphoenol pyruvate synthase, enhancement of transhydrogenase, enhancement of the RhtB gene product, enhancement of the RhtC gene product, enhancement of the YfiK gene product, enhancement of a pyruvate carboxylase, and attenuation of acetic acid formation.

It has been found that microorganisms of the Enterobacteriaceae family produce L-lysine or L-threonine, in particular L-threonine, in an improved manner after over-expression of the rseB gene.

The nucleotide sequences of the genes of Escherichia coli belong to the prior art (see the following text references) and can also be found in the genome sequence of Escherichia coli published by Blattner et al. (Science 277: 1453-1462 (1997)).

The rseB gene or the protein coded by this gene is described, inter alia, by the following data:
- Description:: Regulator RseB of sigma-E factor activity
- Function:: The periplasmic RseB protein regulates sigmaE activity by interaction with periplasmic C-terminal domains of the RseA protein. This imparts an anti-sigmaE activity by inhibition of the transcription of sigmaE-dependent promoters.
- Reference:: Missiakas et al.; Molecular Microbiology 24(2), 355-371 (1997);
De Las Penas et al.; Molecular Microbiology 24(2): 373-385 (1997);
Collinet et al.; Journal of Biological Chemistry 275(43): 33898-33904 (2000)
- Accession No.:: AE000343

The nucleic acid sequences can be found in the databanks of the National Center for Biotechnology Information (NCBI) of the National Library of Medicine (Bethesda, MD, USA), the nucleotide sequence databank of the European Molecular Biologies Laboratories (EMBL, Heidelberg, Germany or Cambridge, UK) or the DNA databank of Japan (DDBJ, Mishima, Japan).

The genes described in the text references mentioned can be used according to the invention. For clarity, the nucleotide sequence of the rseB gene or amino acid sequence of the RseB gene product deposited under Accession No. AE000343 is reproduced as SEQ ID No. 3 and 4. Alleles of the genes which result from the degeneracy of the genetic code or due to at least one (1) "sense mutation" of neutral function can furthermore be used.

Alleles which contain sense mutations of neutral function include, inter alia, those which lead to at least one conservative amino acid exchange in the protein coded by them.

In the case of aromatic amino acids, conservative exchanges are referred to when phenylalanine, tryptophan and tyrosine are exchanged for one another. In the case of hydrophobic amino acids, conservative exchanges are referred to when leucine, isoleucine and valine are exchanged for one another. In the case of polar amino acids, conservative exchanges are referred to when glutamine and asparagine are exchanged for one another. In the case of basic amino acids, conservative exchanges are referred to when arginine, lysine and histidine are exchanged for one another. In the case of acidic amino acids, conservative exchanges are referred to when aspartic acid and glutamic acid are exchanged for one another. In the case of amino acids containing hydroxyl groups, conservative exchanges are referred to when serine and threonine are exchanged for one another.

In the same way, those nucleotide sequences which code for variants of the proteins mentioned which additionally contain a lengthening or shortening by at least one (1) amino acid on the N or C terminus can also be used. This lengthening or shortening is not more than 50, 40, 30, 20, 10, 5, 3 or 2 amino acids or amino acid radicals.

The nucleotide sequence of these genes or alleles or the amino acid sequence of the proteins coded by these genes or alleles is at least 70%, 80%, 85%, 90%, 95%, 96%, 97%, 98% or 99% identical to the sequence of SEQ ID No.3 or 4, the protein having the function of an RseB protein. The use of endogenous genes is preferred.

"Endogenous genes" or "endogenous nucleotide sequences" are understood as meaning the genes or alleles or nucleotide sequences present in the population of a species.

To achieve an over-expression, the number of copies of the corresponding genes can be increased, or the promoter and regulation region or the ribosome binding site upstream of the structural gene can be mutated. Expression cassettes which are incorporated upstream of the structural gene act in the same way. By inducible promoters, it is additionally possible to increase the expression in the course of fermentative L-threonine production. The expression is likewise improved by measures to prolong the life of the m-RNA. Furthermore, the enzyme activity is also increased by preventing the degradation of the enzyme protein. The genes or gene constructs can either be present in plasmids with a varying number of copies, or can be integrated and amplified in the chromosome. Alternatively, an over-expression of the genes in question can furthermore be achieved by changing the composition of the media and the culture procedure.

The expert can find instructions in this respect, inter alia, in Chang and Cohen (Journal of Bacteriology 134: 1141-1156 (1978)), in Hartley and Gregori (Gene 13: 347-353 (1981)), in Amann and Brosius (Gene 40: 183-190 (1985)), in de Broer et al. (Proceedings of the National Academy of Sciences of the United States of America 80: 21-25 (1983)), in LaVallie et al. (BIO/TECHNOLOGY 11: 187-193 (1993)), in PCT/US97/13359, in Llosa et al. (Plasmid 26: 222-224 (1991)), in Quandt and Klipp (Gene 80: 161-169 (1989)), in Hamilton et al. (Journal of Bacteriology 171: 4617-4622 (1989)), in Jensen and Hammer (Biotechnology and Bioengineering 58: 191-195 (1998)) and in known textbooks of genetics and molecular biology.

Plasmid vectors which can replicate in Enterobacteriaceae, such as e.g. cloning vectors derived from pACYC184 (Bartolome et al.; Gene 102: 75-78 (1991)), pTrc99A (Amann et al.; Gene 69: 301-315 (1988)) or pSC101 derivatives (Vocke and Bastia; Proceedings of the National Academy of Sciences USA 80(21): 6557-6561 (1983)) can be used. A strain transformed with a plasmid vector where the plasmid vector carries at least one nucleotide sequence which codes for the rseB gene can be employed in a process according to the invention.

The term transformation is understood in general as meaning the uptake of an isolated nucleic acid by a host (microorganism).

It is also possible to transfer mutations which affect the expression of the particular genes into various strains by sequence exchange (Hamilton et al.; Journal of Bacteriology 171: 4617-4622 (1989)), conjugation or transduction.

More detailed explanations of terms in genetics and molelcular biology are found in known textbooks of genetics and molecular biology, such as, for example, the textbook by Birge (Bacterial and Bacteriophage Genetics, 4th ed., Springer Verlag, New York (USA), 2000) or the textbook by Stryer (Biochemistry, 3rd ed., Freeman and Company, New York (USA), 1988) or the handbook by Sambrook et al. (Molecular Cloning, A Laboratory Manual, 2nd ed., Cold Spring Harbor Laboratory Press, Cold Spring Harbor (USA), 1989).

It may furthermore be advantageous for the production of L-lysine or L-threonine with strains of the Enterobacteriaceae family to enhance one or more enzymes of the known threonine biosynthesis pathway or enzymes of anaplerotic metabolism or enzymes for the production of reduced nicotinamide adenine dinucleotide phosphate or enzymes of glycolysis or PTS enzymes or enzymes of sulfur metabolism, in addition to the enhancement of the rseB gene. The use of endogenous genes is in general preferred.

Thus, for example, at the same time one or more of the genes chosen from the group consisting of
- the thrABC operon which codes for aspartate kinase, homoserine dehydrogenase, homoserine kinase and threonine synthase (US-A-4,278,765),
- the pyc gene of Corynebacterium glutamicum which codes for pyruvate carboxylase (WO 99/18228),
- the pps gene which codes for phosphoenol pyruvate synthase (Molecular and General Genetics 231(2): 332-336 (1992)),
- the ppc gene which codes for phosphoenol pyruvate carboxylase (Gene 31: 279-283 (1984)),
- the pntA and pntB genes which code for transhydrogenase (European Journal of Biochemistry 158: 647-653 (1986)),
- the rhtB gene which imparts homoserine resistance (EP-A-0 994 190),
- the mqo gene which codes for malate:quinone oxidoreductase (WO 02/06459),
- the rhtC gene which imparts threonine resistance (EP-A-1 013 765),
- the thrE gene of Corynebacterium glutamicum which codes for the threonine export protein (WO 01/92545),
- the gdhA gene which codes for glutamate dehydrogenase (Nucleic Acids Research 11: 5257-5266 (1983); Gene 23: 199-209 (1983)),
- the hns gene which codes for the DNA-binding protein HLP-II (WO 03/004671),
- the pgm gene which codes for phosphoglucomutase (WO 03/004598),
- the fba gene which codes for fructose biphosphate aldolase (WO 03/004664),
- the ptsH gene of the ptsHIcrr operon which codes for the phosphohistidine protein hexose phosphotransferase of the phosphotransferase system PTS (WO 03/004674),
- the ptsI gene of the ptsHIcrr operon which codes for enzyme I of the phosphotransferase system PTS (WO 03/004674),
- the crr gene of the ptsHIcrr operon which codes for the glucose-specific IIA component of the phosphotransferase system PTS (WO 03/004674),
- the ptsG gene which codes for the glucose-specific IIBC component (WO 03/004670),
- the lrp gene which codes for the regulator of the leucine regulon (WO 03/004665),
- the csrA gene which codes for the global regulator Csr (Journal of Bacteriology 175: 4744-4755 (1993)),
- the fadR gene which codes for the regulator of the fad regulon (Nucleic Acids Research 16: 7995-8009 (1988)),
- the iclR gene which codes for the regulator of the central intermediate metabolism (Journal of Bacteriology 172: 2642-2649 (1990)),
- the mopB gene which codes for the 10 Kd chaperone (WO 03/004669) and is also known by the name groES,
- the ahpC gene of the ahpCF operon which codes for the small sub-unit of alkyl hydroperoxide reductase (WO 03/004663),
- the ahpF gene of the ahpCF operon which codes for the large sub-unit of alkyl hydroperoxide reductase (WO 03/004663),
- the cysK gene which codes for cysteine synthase A (WO 03/006666),
- the cysB gene which codes for the regulator of the cys regulon (WO 03/006666),
- the cysJ gene of the cysJIH operon which codes for the flavoprotein of NADPH sulfite reductase (WO 03/006666),
- the cysI gene of the cysJIH operon which codes for the haemoprotein of NADPH sulfite reductase (WO 03/006666),
- the cysH gene of the cysJIH operon which codes for adenylyl sulfate reductase (WO 03/006666),
- the phoB gene of the phoBR operon which codes for the positive regulator PhoB of the pho regulon (WO 03/008606),
- the phoR gene of the phoBR operon which codes for the sensor protein of the pho regulon (WO 03/008606),
- the phoE gene which codes for protein E of the outer cell membrane (WO 03/008608),
- the pykF gene which codes for fructose-stimulated pyruvate kinase I (WO 03/008609),
- the pfkB gene which codes for 6-phosphofructokinase II (WO 03/008610),
- the malE gene which codes for the periplasmic binding protein of maltose transport (WO 03/008605),
- the sodA gene which codes for superoxide dismutase (WO 03/008613),
- the rseA gene of the rseABC operon which codes for a membrane protein with anti-sigmaE activity (WO 03/008612),
- the rseC gene of the rseABC operon which codes for a global regulator of the sigmaE factor (WO 03/008612),
- the sucA gene of the sucABCD operon which codes for the decarboxylase sub-unit of 2-ketoglutarate dehydrogenase (WO 03/008614),
- the sucB gene of the sucABCD operon which codes for the dihydrolipoyltranssuccinase E2 sub-unit of 2-ketoglutarate dehydrogenase (WO 03/008614),
- the sucC gene of the sucABCD operon which codes for the β-sub-unit of succinyl-CoA synthetase (WO 03/008615),
- the sucD gene of the sucABCD operon which codes for the α-sub-unit of succinyl-CoA synthetase (WO 03/008615),
- the adk gene which codes for adenylate kinase (Nucleic Acids Research 13(19): 7139-7151 (1985)),
- the hdeA gene which codes for a periplasmic protein with a chaperonin-like function (Journal of Bacteriology 175(23): 7747-7748 (1993)),
- the hdeB gene which codes for a periplasmic protein with a chaperonin-like function (Journal of Bacteriology 175(23): 7747-7748 (1993)),
- the icd gene which codes for isocitrate dehydrogenase (Journal of Biological Chemistry 262(22): 10422-10425 (1987)),
- the mglB gene which codes for the periplasmic, galactose-binding transport protein (Molecular and General Genetics 229(3): 453-459 (1991)),
- the lpd gene which codes for dihydrolipoamide dehydrogenase (European Journal of Biochemistry 135(3): 519-527 (1983)),
- the aceE gene which codes for the E1 component of the pyruvate dehydrogenase complex (European Journal of Biochemistry 133 (1) : 155-162 (1983)),
- the aceF gene which codes for the E2 component of the pyruvate dehydrogenase complex (European Journal of Biochemistry 133(3): 481-489 (1983)),
- the pepB gene which codes for aminopeptidase B (Journal of Fermentation and Bioengineering 82: 392-397 (1996)) and
- the aldH gene which codes for aldehyde dehydrogenase (E.C. 1.2.1.3) (Gene 99(1): 15-23 (1991)),
can be over-expressed.

It may furthermore be advantageous for the production of L-lysine or L-threonine, in addition to the overexpression of the rseB gene, for one or more of the genes chosen from the group consisting of
- the tdh gene which codes for threonine dehydrogenase (Journal of Bacteriology 169: 4716-4721 (1987)),
- the mdh gene which codes for malate dehydrogenase (E.C. 1.1.1.37) (Archives in Microbiology 149: 36-42 (1987)),
- the gene product of the open reading frame (orf) yjfA (Accession Number AAC77180 of the National Center for Biotechnology Information (NCBI, Bethesda, MD, USA), WO 02/29080),
- the gene product of the open reading frame (orf) ytfP (Accession Number AAC77179 of the National Center for Biotechnology Information (NCBI, Bethesda, MD, USA), (WO 02/29080),
- the pckA gene which codes for the enzyme phosphoenol pyruvate carboxykinase (WO 02/29080),
- the poxB gene which codes for pyruvate oxidase (WO 02/36797),
- the aceA gene which codes for the enzyme isocitrate lyase (WO 02/081722),
- the dgsA gene which codes for the DgsA regulator of the phosphotransferase system (WO 02/081721) and is also known under the name of the mlc gene,
- the fruR gene which codes for the fructose repressor (WO 02/081698) and is also known under the name of the cra gene,
- the rpoS gene which codes for the sigma³⁸ factor (WO 01/05939) and is also known under the name of the katF gene,
- the aspA gene which codes for aspartate ammonium lyase (WO 03/008603) and
- the aceB gene which codes for malate synthase A (WO 03/008604)
to be eliminated.

The term "attenuation" in this connection describes the reduction or elimination of the intracellular activity or concentration of one or more enzymes or proteins in a microorganism which are coded by the corresponding DNA, for example by using a weak promoter or a gene or allele which codes for a corresponding enzyme or protein with a low activity or inactivates the corresponding enzyme (protein) or gene and optionally combining these measures.

By attenuation measures, the activity or concentration of the corresponding protein is in general reduced to 0 to 75%, 0 to 50%, 0 to 25%, 0 to 10% or 0 to 5% of the activity or concentration of the wild-type protein or of the activity or concentration of the protein in the starting microorganism.

In addition to overexpression of the rseB gene it may furthermore be advantageous for the production of L-amino acids, in particular L-threonine, to eliminate undesirable side reactions (Nakayama: "Breeding of Amino Acid Producing Microorganisms", in: Overproduction of Microbial Products, Krumphanzl, Sikyta, Vanek (eds.), Academic Press, London, UK, 1982).

The microorganisms produced according to the invention can be cultured in the batch process (batch culture), the fed batch (feed process) or the repeated fed batch process (repetitive feed process). A summary of known culture methods is described in the textbook by Chmiel (Bioprozesstechnik 1. Einfuhrung in die Bioverfahrenstechnik (Gustav Fischer Verlag, Stuttgart, 1991)) or in the textbook by Storhas (Bioreaktoren und periphere Einrichtungen (Vieweg Verlag, Braunschweig/Wiesbaden, 1994)).

The culture medium to be used must meet the requirements of the particular strains in a suitable manner. Descriptions of culture media for various microorganisms are contained in the handbook "Manual of Methods for General Bacteriology" of the American Society for Bacteriology (Washington D.C., USA, 1981).

Sugars and carbohydrates, such as e.g. glucose, sucrose, lactose, fructose, maltose, molasses, starch and optionally cellulose, oils and fats, such as e.g. soya oil, sunflower oil, groundnut oil and coconut fat, fatty acids, such as e.g. palmitic acid, stearic acid and linoleic acid, alcohols, such as e.g. glycerol and ethanol, and organic acids, such as e.g. acetic acid, can be used as the source of carbon. These substances can be used individually or as a mixture.

Organic nitrogen-containing compounds, such as peptones, yeast extract, meat extract, malt extract, corn steep liquor, soya bean flour and urea, or inorganic compounds, such as ammonium sulfate, ammonium chloride, ammonium phosphate, ammonium carbonate and ammonium nitrate, can be used as the source of nitrogen. The sources of nitrogen can be used individually or as a mixture.

Phosphoric acid, potassium dihydrogen phosphate or dipotassium hydrogen phosphate or the corresponding sodium-containing salts can be used as the source of phosphorus. The culture medium must furthermore comprise salts of metals, such as e.g. magnesium sulfate or iron sulfate, which are necessary for growth. Finally, essential growth substances, such as amino acids and vitamins, can be employed in addition to the abovementioned substances.

Suitable precursors can moreover be added to the culture medium. The starting substances mentioned can be added to the culture in the form of a single batch, or can be fed in during the culturing in a suitable manner.

The fermentation is in general carried out at a pH of 5.5 to 9.0, in particular 6.0 to 8.0. Basic compounds, such as sodium hydroxide, potassium hydroxide, ammonia or aqueous ammonia, or acid compounds, such as phosphoric acid or sulfuric acid, can be employed in a suitable manner to control the pH of the culture. Antifoams, such as e.g. fatty acid polyglycol esters, can be employed to control the development of foam. Suitable substances having a selective action, e.g. antibiotics, can be added to the medium to maintain the stability of plasmids. To maintain aerobic conditions, oxygen or oxygen-containing gas mixtures, such as e.g. air, are introduced into the culture. The temperature of the culture is usually 25°C to 45°C, and preferably 30°C to 40°C. Culturing is continued until a maximum of L-amino acids or L-threonine has formed. This target is usually reached within 10 hours to 160 hours.

The analysis of L-amino acids can be carried out by anion exchange chromatography with subsequent ninhydrin derivatization, as described by Spackman et al. (Analytical Chemistry, 30: 1190-1206 (1958)) or it can be carried out by reversed phase HPLC, as described by Lindroth et al. (Analytical Chemistry 51: 1167-1174 (1979)).

The process according to the invention is used for the fermentative preparation of L-threonine or L-lysine, in particular L-threonine.

The present invention is explained in more detail in the following with the aid of embodiment examples.

The minimal (M9) and complete media (LB) for Escherichia coli used are described by J.H. Miller (A short course in bacterial genetics (1992), Cold Spring Harbor Laboratory Press). The isolation of plasmid DNA from Escherichia coli and all techniques of restriction, ligation, Klenow and alkaline phosphatase treatment are carried out by the method of Sambrook et al. (Molecular Cloning - A Laboratory Manual (1989) Cold Spring Harbor Laboratory Press). Unless described otherwise, the transformation of Escherichia coli is carried out by the method of Chung et al. (Proceedings of the National Academy of Sciences of the United States of America 86: 2172-2175 (1989)).

The incubation temperature for the preparation of strains and transformants is 37ºC.

### Example 1

### Construction of the expression plasmid pTrc99ArseB

The rseB gene from E. coli K12 is amplified using the polymerase chain reaction (PCR) and synthetic oligonucleotides. Starting from the nucleotide sequence of the rseB gene in E. coli K12 MG1655 (Accession Number AE000343, Blattner et al. (Science 277: 1453-1474 (1997)), PCR primers are synthesized (MWG Biotech, Ebersberg, Germany).
rseB1:
   5' - GATAGCGGGATTCTAGATAAGGGTATTAGG - 3'(SEQ ID No. 1)
rseB2:
   5' - GCAACAACTGCAGTGAAATCACTGG - 3' (SEQ ID No. 2)

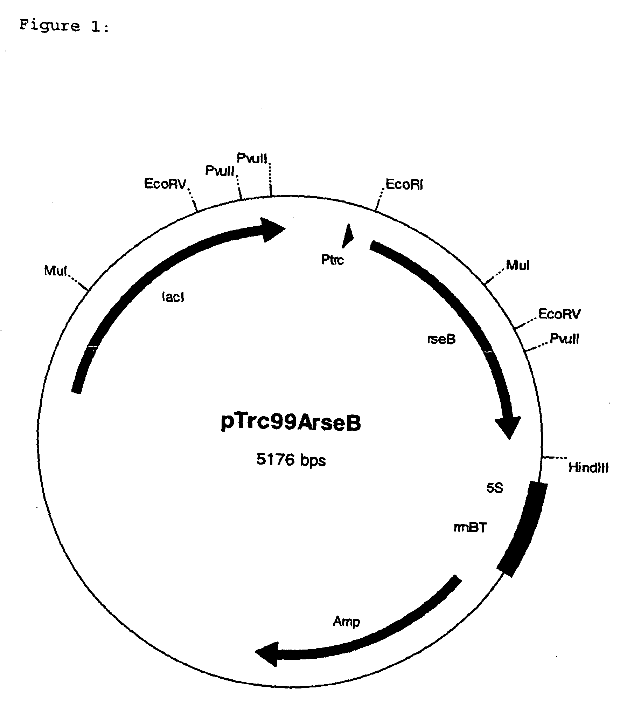

The chromosomal E. coli K12 MG1655 DNA employed for the PCR is isolated according to the manufacturer's instructions with "Qiagen Genomic-tips 100/G" (QIAGEN, Hilden, Germany). A DNA fragment approx. 2100 bp in size can be amplified with the specific primers under standard PCR conditions (Innis et al. (1990) PCR Protocols. A guide to methods and applications, Academic Press) with Pfu-DNA polymerase (Promega Corporation, Madison, USA). The PCR product is cleaved with the restriction enzymes EcoRI and HindIII. After separation in 0.8% agarose gel a fragment approx. 1,000 bp in size is isolated and ligated with the vector pTrc99A (Pharmacia Biotech, Uppsala, Sweden), which has been digested with the enzymes EcoRI and HindIII. The E. coli strain XL1-Blue MRF' (Stratagene, La Jolla, USA) is transformed with the ligation batch and plasmid-carrying cells are selected on LB agar, to which 50 µg/ml ampicillin are added. Successful cloning can be demonstrated after plasmid DNA isolation by control cleavage with the enzymes EcoRV, MluI and PvuII. The plasmid is called pTrc99ArseB (figure 1).

### Example 2

### Preparation of L-threonine with the strain MG442/pTrc99ArseB

The L-threonine-producing E. coli strain MG442 is described in the patent specification US-A-4,278,765 and deposited as CMIM B-1628 at the Russian National Collection for Industrial Microorganisms (VKPM, Moscow, Russia).

The strain MG442 is transformed with the expression plasmid pTrc99ArseB described in example 1 and with the vector pTrc99A and plasmid-carrying cells are selected on LB agar with 50 µg/ml ampicillin. The strains MG442/pTrc99ArseB and MG442/pTrc99A are formed in this manner. Selected individual colonies are then multiplied further on minimal medium with the following composition: 3.5 g/l Na₂HPO₄*2H₂O, 1.5 g/l KH₂PO₄, 1 g/l NH₄Cl, 0.1 g/l MgSO₄*7H₂O, 2 g/l glucose, 20 g/l agar, 50 mg/l ampicillin. The formation of L-threonine is checked in batch cultures of 10 ml contained in 100 ml conical flasks. For this, 10 ml of preculture medium of the following composition: 2 g/l yeast extract, 10 g/l (NH₄)₂SO₄, 1 g/l KH₂PO₄, 0.5 g/l MgSO₄*7H₂O, 15 g/l CaCO₃, 20 g/l glucose, 50 mg/l ampicillin are inoculated and the batch is incubated for 16 hours at 37°C and 180 rpm on an ESR incubator from Kühner AG (Birsfelden, Switzerland). 250 µl portions of this preculture are transinoculated into 10 ml of production medium (25 g/l (NH₄)₂SO₄, 2 g/l KH₂PO₄, 1 g/l MgSO₄*7H₂O, 0.03 g/l FeSO₄*7H₂O, 0.018 g/l MnSO₄*1H₂O, 30 g/l CaCO₃, 20 g/l glucose, 50 mg/l ampicillin) and the batch is incubated for 48 hours at 37ºC. The formation of L-threonine by the starting strain MG442 is investigated in the same manner, but no addition of ampicillin to the medium takes place. After the incubation the optical density (OD) of the culture suspension is determined with an LP2W photometer from Dr. Lange (Düsseldorf, Germany) at a measurement wavelength of 660 nm.

The concentration of L-threonine formed is then determined in the sterile-filtered culture supernatant with an amino acid analyzer from Eppendorf-BioTronik (Hamburg, Germany) by ion exchange chromatography and post-column reaction with ninhydrin detection.

The result of the experiment is shown in table 1.

**Table 1**

| Strain | OD | L-Threonine g/l |
|---|---|---|
| MG442 | 5.6 | 1.4 |
| MG442/pTrc99A | 3.8 | 1.3 |
| MG442/pTrc99ArseB | 5.7 | 2.3 |

### Brief description of the figure:

Figure 1: Map of the plasmid pTrc99ArseB containing the rseB gene.

The length data are to be understood as approx. data. The abbreviations and designations used have the following meaning:
- Amp: Ampicillin resistance gene
- lacI:Gene for the repressor protein of the trc promoter
- Ptrc: trc promoter region, IPTG-inducible
- rseB: Coding region of the rseB gene
- 5S:5S rRNA region
- rrnBT: rRNA terminator region

The abbreviations for the restriction enzymes have the following meaning
- EcoRI: Restriction endonuclease from Escherichia coli RY13
- ECORV: Restriction endonuclease from Escherichia coli B946
- HindIII: Restriction endonuclease from Haemophilus influenzae
- MluI: Restriction endonuclease from Micrococcus luteus IFO 12992
- PvuII: Restriction endonuclease from Proteus vulgaris (ATCC 13315)

## Claims

1. Process for the preparation of L-amino acids, chosen from the group L-threonine and L-lysine, or feedstuffs additives comprising these compounds by fermentation of microorganisms of the Enterobacteriaceae family, wherein the rseB gene, coding for the regulator of sigma-E factor activity, or nucleotide sequences which code for the rseB gene product is or are over-expressed, and isolation of said L-amino acid.

2. Process according to claim 1, **characterized in that** recombinant microorganisms which are generated by transformation, transduction or conjugation of a microorganism of the Enterobacteriaceae family with a vector, wherein the vector contains an rseB gene, are employed.

3. Process according to claim 1, **characterized in that** in the recombinant microorganisms the number of copies of the rseB gene is increased by at least 1.

4. Process according to claim 3, **characterized in that** in the recombinant microorganisms the increase in the number of copies of the rseB gene by at least 1 is achieved by integration of the gene into the chromosome of the microorganism.

5. Process according to claim 3, **characterized in that** in the recombinant microorganisms the increase in the number of copies of the rseB gene by at least 1 is achieved by the use of a vector which replicates extrachromosomally.

6. Process according to claim 1, **characterized in that** the rseB gene placed under the control of a promoter is used.

7. Process according to claim 1, **characterized in that** microorganisms chosen from the genera Escherichia, Erwinia, Providencia and Serratia are employed.

8. Process according to claim 1, **characterized in that**, for the preparation of L-threonine microorganisms of the Enterobacteriaceae family in which additionally at the same time one or more of the genes chosen from the group consisting of:
8.1 the thrABC operon which codes for aspartate kinase, homoserine dehydrogenase, homoserine kinase and threonine synthase,
8.2 the pyc gene which codes for pyruvate carboxylase,
8.3 the pps gene which codes for phosphoenol pyruvate synthase,
8.4 the ppc gene which codes for phosphoenol pyruvate carboxylase,
8.5 the pntA and pntB genes which code for transhydrogenase,
8.6 the Escherichia coli rhtB gene coding for a protein imparting homoserine resistance,
8.7 the mqo gene which codes for malate:quinone oxidoreductase,
8.8 the Escherichia coli rhtC gene coding for a protein imparting threonine resistance,
8.9 the thrE gene which codes for the threonine export protein,
8.10 the gdhA gene which codes for glutamate dehydrogenase,
8.11 the hns gene which codes for the DNA-binding protein HLP-II,
8.12 the pgm gene which codes for phosphoglucomutase,
8.13 the fba gene which codes for fructose biphosphate aldolase,
8.14 the ptsH gene which codes for the phosphohistidine protein hexose phosphotransferase,
8.15 the ptsI gene which codes for enzyme I of the phosphotransferase system,
8.16 the crr gene which codes for the glucose-specific IIA component,
8.17 the ptsG gene which codes for the glucose-specific IIBC component,
8.18 the lrp gene which codes for the regulator of the leucine regulon,
8.19 the csrA gene which codes for the global regulator Csr,
8.20 the fadR gene which codes for the regulator of the fad regulon,
8.21 the iclR gene which codes for the regulator of central intermediate metabolism,
8.22 the mopB gene which codes for the 10 Kd chaperone,
8.23 the ahpC gene which codes for the small sub-unit of alkyl hydroperoxide reductase,
8.24 the ahpF gene which codes for the large sub-unit of alkyl hydroperoxide reductase,
8.25 the cysK gene which codes for cysteine synthase A,
8.26 the cysB gene which codes for the regulator of the cys regulon,
8.27 the cysJ gene which codes for the flavoprotein of NADPH sulfite reductase,
8.28 the cysI gene which codes for the haemoprotein of NADPH sulfite reductase,
8.29 the cysH gene which codes for adenylyl sulfate reductase,
8.30 the phoB gene which codes for the positive regulator PhoB of the pho regulon,
8.31 the phoR gene which codes for the sensor protein of the pho regulon,
8.32 the phoE gene which codes for protein E of the outer cell membrane,
8.33 the pykF gene which codes for fructose-stimulated pyruvate kinase I,
8.34 the pfkB gene which codes for 6-phosphofructokinase II,
8.35 the malE gene which codes for the periplasmic binding protein of maltose transport,
8.36 the sodA gene which codes for superoxide dismutase,
8.37 the rseA gene which codes for a membrane protein with anti-sigmaE activity,
8.38 the rseC gene which codes for a global regulator of the sigmaE factor
8.39 the sucA gene which codes for the decarboxylase sub-unit of 2-ketoglutarate dehydrogenase,
8.40 the sucB gene which codes for the dihydrolipoyltranssuccinase E2 sub-unit of 2-ketoglutarate dehydrogenase,
8.41 the sucC gene which codes for the β-sub-unit of succinyl-CoA synthetase,
8.42 the sucD gene which codes for the α-sub-unit of succinyl-CoA synthetase,
8.43 the adk gene which codes for adenylate kinase,
8.44 the hdeA gene which codes for a periplasmic protein with a chaperonin-like function,
8.45 the hdeB gene which codes for a periplasmic protein with a chaperonin-like function,
8.46 the icd gene which codes for isocitrate dehydrogenase,
8.47 the mglB gene which codes for the periplasmic, galactose-binding transport protein,
8.48 the lpd gene which codes for dihydrolipoamide dehydrogenase,
8.49 the aceE gene which codes for the E1 component of the pyruvate dehydrogenase complex,
8.50 the aceF gene which codes for the E2 component of the pyruvate dehydrogenase complex,
8.51 the pepB gene which codes for aminopeptidase B and
8.52 the aldH gene which codes for aldehyde dehydrogenase,
is or are over-expressed, are fermented.

9. Process according to claim 1 or 8, **characterized in that**, for the preparation of L-threonine, microorganisms of the Enterobacteriaceae family in which additionally at the same time one or more of the genes chosen from the group consisting of:
9.1 the tdh gene which codes for threonine dehydrogenase,
9.2 the mdh gene which codes for malate dehydrogenase,
9.3 the gene product of the open reading frame (orf) yjfA of E. coli, when said microorganism is Escherichia coli,
9.4 the gene product of the open reading frame (orf) ytfP of E. coli, when said microorganism is Escherichia coli"
9.5 the pckA gene which codes for phosphoenol pyruvate carboxykinase,
9.6 the poxB gene which codes for pyruvate oxidase,
9.7 the aceA gene which codes for isocitrate lyase,
9.8 the dgsA gene which codes for the DgsA regulator of the phosphotransferase system,
9.9 the fruR gene which codes for the fructose repressor,
9.10 the rpoS gene which codes for the sigma³⁸ factor,
9.11 the aspA gene which codes for aspartate ammonium lyase and
9.12 the aceB gene which codes for malate synthase A
is or are eliminated are fermented.

10. Process according to one or more of the claims 1 to 9, in which
a) the desired L-amino acid is concentrated in the fermentation broth or in the cells of the microorganisms, and
b) the product(s) containing the desired L-amino acid is/are isolated, the biomass and/or further constituents of the fermentation broth optionally remaining in the product in an amount of > 10 to 100 %.

11. Recombinant microorganisms of the Enterobacteriaceae family, which produce L-threonine, and wherein at least:
a) the thrABC operon which genes code for aspartate kinase, homoserine dehydrogenase, homoserine kinase and threonine synthase, and
b) the rseB gene, coding for the regulator of sigma-E factor activity, or nucleotide sequences which code for the rseB gene product,
are over-expressed.

12. Recombinant microorganisms of the Enterobacteriaceae family, which produce L-lysine, and wherein at least:
a) the pckA gene coding for the phosphoenol pyruvate carboxykinase is eliminated, and
b) the rseB gene, coding for the regulator of sigma-E factor activity, or nucleotide sequences which code for the rseB gene product is or are over-expressed.

13. Microorganisms according to claim 11 to 12, wherein the microorganisms originate from the genus Escherichia.

14. Microorganisms according to claim 13, wherein the microorganisms originate from the species E. coli.

## Patentansprüche

1. Verfahren zur Herstellung von L-Aminosäuren, ausgewählt aus der Gruppe L-Threonin und L-Lysin, oder von Futteradditiven, die diese Verbindungen umfassen, durch Fermentation von Mikroorganismen der Familie Enterobacteriaceae, in denen man das rseB-Gen, das für den Regulator der SigmaE-Faktor-Aktivität kodiert, oder für das rseB-Genprodukt kodierende Nukleotidsequenzen überexprimiert, und Isolierung der L-Aminosäure.

2. Verfahren nach Anspruch 1, **dadurch gekennzeichnet, dass** man rekombinante Mikroorganismen einsetzt, die durch Transformation, Transduktion oder Konjugation eines Mikroorganismus der Familie Enterobacteriaceae mit einem Vektor erzeugt werden, wobei der Vektor ein rseB-Gen enthält.

3. Verfahren nach Anspruch 1, **dadurch gekennzeichnet, dass** man in den rekombinanten Mikroorganismen die Anzahl der Kopien des rseB-gens um mindestens 1 erhöht.

4. Verfahren nach Anspruch 3, **dadurch gekennzeichnet, dass** man in den rekombinanten Mikroorganismen die Erhöhung der Anzahl der Kopien des rseB-Gens um mindestens 1 durch Integration des Gens in das Chromosom des Mikroorganismus erzielt.

5. Verfahren nach Anspruch 3, **dadurch gekennzeichnet, dass** man in den rekombinanten Mikroorganismen die Erhöhung der Anzahl der Kopien des rseB-Gens um mindestens 1 durch Verwendung eines sich extrachromosomal replizierenden Vektors erzielt.

6. Verfahren nach Anspruch 1, **dadurch gekennzeichnet, dass** man das unter der Kontrolle eines Promotors stehende rseB-Gen verwendet.

7. Verfahren nach Anspruch 1, **dadurch gekennzeichnet, dass** man aus den Gattungen Escherichia, Erwinia, Providencia und Serratia ausgewählte Mikroorganismen einsetzt.

8. Verfahren nach Anspruch 1, **dadurch gekennzeichnet, dass** man zur Herstellung von L-Threonin Mikroorganismen der Familie Enterobacteriaceae fermentiert, in denen man zusätzlich gleichzeitig eines oder mehrere der Gene, ausgewählt aus der Gruppe, bestehend aus:
8.1 dem für die Aspartatkinase, die Homoserindehydrogenase, die Homoserinkinase und die Threoninsynthase kodierenden thrABC-Operon,
8.2 dem für die Pyruvat-Carboxylase kodierenden pyc-Gen,
8.3 dem für die Phosphoenolpyruvat-Synthase kodierenden pps-Gen,
8.4 dem für die Phosphoenolpyruvat-Carboxylase kodierenden ppc-Gen,
8.5 den für die Transhydrogenase kodierenden Genen pntA und pntB,
8.6 dem rhtB-Gen von Escherichia coli, das für ein Protein kodiert, das Homoserinresistenz vermittelt,
8.7 dem für die Malat:Chinon-Oxidoreduktase kodierenden mqo-Gen,
8.8 dem rhtC-Gen von Escherichia coli, das für ein Protein kodiert, das Threoninresistenz vermittelt,
8.9 dem für das Threonin-Exportprotein kodierenden thrE-Gen,
8.10 dem für die Glutamat-Dehydrogenase kodierenden gdhA-Gen,
8.11 dem für das DNA-Bindeprotein HLP-II kodierenden hns-Gen,
8.12 dem für die Phosphoglucomutase kodierenden pgm-Gen,
8.13 dem für die Fructosebisphosphat-Aldolase kodierenden fba-Gen,
8.14 dem für die Phosphohistidin-Protein-Hexose-Phosphotransferase kodierenden ptsH-Gen,
8.15 dem für das Enzym I des Phosphotransferase-Systems kodierenden ptsI-Gen,
8.16 dem für die Glucose-spezifische IIA-Komponente kodierenden crr-Gen,
8.17 dem für die Glucose-spezifische IIBC-Komponente kodierenden ptsG-Gen,
8.18 dem für den Regulator des Leucin-Regulons kodierenden lrp-Gen,
8.19 dem für den globalen Regulator Csr kodierenden csrA-Gen,
8.20 dem für den Regulator des fad-Regulons kodierenden fadR-Gen,
8.21 dem für den Regulator des zentralen Intermediärstoffwechsels kodierenden iclR-Gen,
8.22 dem für das 10 Kd-Chaperon kodierenden mopB-Gen,
8.23 dem für die kleine Untereinheit der Alkylhydroperoxid-Reduktase kodierenden ahpC-Gen,
8.24 dem für die große Untereinheit der Alkylhydroperoxid-Reduktase kodierenden ahpF-Gen,
8.25 dem für die Cystein-Synthase A kodierenden cysK-Gen,
8.26 dem für den Regulator des cys-Regulons kodierenden cysB-Gen,
8.27 dem für das Flavoprotein der NADPH-Sulfit-Reduktase kodierenden cysJ-Gen,
8.28 dem für das Hämoprotein der NADPH-Sulfit-Reduktase kodierenden cysI-Gen,
8.29 dem für die Adenylylsulfat-Reduktase kodierenden cysH-Gen,
8.30 dem für den positiven Regulator PhoB des pho-Regulons kodierenden phoB-Gen,
8.31 dem für das Sensorprotein des pho-Regulons kodierenden phoR-Gen,
8.32 dem für das Protein E der äußeren Zellmembran kodierenden phoE-Gen,
8.33 dem für die Fructose-stimulierte Pyruvatkinase I kodierenden pykF-Gen,
8.34 dem für die 6-Phosphofructokinase II kodierenden pfkB-Gen,
8.35 dem für das periplasmatische Bindungsprotein des Maltosetransports kodierenden malE-Gen,
8.36 dem für die Superoxiddismutase kodierenden sodA-Gen,
8.37 dem für ein Membranprotein mit Anti-SigmaE-Aktivität kodierenden rseA-Gen,
8.38 dem für einen globalen Regulator des SigmaE-Faktors kodierenden rseC-Gen,
8.39 dem für die Decarboxylase-Untereinheit der 2-Ketoglutarat-Dehydrogenase kodierenden sucA-Gen,
8.40 dem für die Dihydrolipoyltranssuccinase-E2-Untereinheit der 2-Ketoglutarat-Dehydrogenase kodierenden sucB-Gen,
8.41 dem für die β-Untereinheit der Succinyl-CoA-Synthetase kodierenden sucC-Gen,
8.42 dem für die α-Untereinheit der Succinyl-CoA-Synthetase kodierenden sucD-Gen,
8.43 dem für die Adenylatkinase kodierenden adk-Gen,
8.44 dem für ein periplasmatisches Protein mit einer Chaperonin-ähnlichen Funktion kodierenden hdeA-Gen,
8.45 dem für ein periplasmatisches Protein mit einer Chaperonin-ähnlichen Funktion kodierenden hdeB-Gen,
8.46 dem für die Isocitrat-Dehydrogenase kodierenden icd-Gen,
8.47 dem für das periplasmatische Galactose-Bindungs-Transportprotein kodierenden mglB-Gen,
8.48 dem für die Dihydrolipoamid-Dehydrogenase kodierenden lpd-Gen,
8.49 dem für die E1-Komponente des Pyruvatdehydrogenase-Komplexes kodierenden aceE-Gen,
8.50 dem für die E2-Komponente des Pyruvatdehydrogenase-Komplexes kodierenden aceF-Gen,
8.51 dem für die Aminopeptidase B kodierenden pepB-Gen und
8.52 dem für die Aldehyd-Dehydrogenase kodierenden aldH-Gen,
überexprimiert.

9. Verfahren nach Anspruch 1 oder 8, **dadurch gekennzeichnet, dass** man zur Herstellung von L-Threonin Mikroorganismen der Familie Enterobacteriaceae fermentiert, in denen man zusätzlich gleichzeitig eines oder mehrere der Gene, ausgewählt aus der Gruppe, bestehend aus:
9.1 dem für die Threonin-Dehydrogenase kodierenden tdh-Gen,
9.2 dem für die Malat-Dehydrogenase kodierenden mdh-Gen,
9.3 dem Genprodukt des offenen Leserahmens (orf) yjfA von E. coli, wenn es sich bei dem Mikroorganismus um Escherichia coli handelt,
9.4 dem Genprodukt des offenen Leserahmens (orf) ytfP von E. coli, wenn es sich bei dem Mikroorganismus um Escherichia coli handelt,
9.5 dem für die Phosphoenolpyruvat-Carboxykinase kodierenden pckA-Gen,
9.6 dem für die Pyruvat-Oxidase kodierenden poxB-Gen,
9.7 dem für die Isocitrat-Lyase kodierenden aceA-Gen,
9.8 dem für den DgsA-Regulator des Phosphotransferase-Systems kodierenden dgsA-Gen,
9.9 dem für den Fructose-Repressor kodierenden fruR-Gen,
9.10 dem für den Sigma³⁸-Faktor kodierenden rpoS-Gen,
9.11 dem für die Aspartat-Ammonium-Lyase kodierenden aspA-Gen und
9.12 dem für die Malatsynthase A kodierenden aceB-Gen, ausschaltet.

10. Verfahren nach einem oder mehreren der Ansprüche 1 bis 9, wobei man
a) die gewünschte L-Aminosäure in der Fermentationsbrühe oder in den Zellen der Mikroorganismen konzentriert und
b) das (die) die gewünschte L-Aminosäure enthaltende(n) Produkt(e) isoliert, wobei die Biomasse und/oder weitere Bestandteile der Fermentationsbrühe gegebenenfalls in dem Produkt in einer Menge von > 0 bis 100% verbleiben.

11. Rekombinante Mikroorganismen aus der Familie Enterobacteriaceae, die L-Threonin produzieren und wobei man mindestens:
a) das thrABC-Operon, dessen Gene für die Aspartatkinase, die Homoserindehydrogenase, die Homoserinkinase und die Threoninsynthase kodieren, und
b) das rseB-Gen, das für den Regulator der SigmaE-Faktor-Aktivität kodiert, oder für das rseB-Genprodukt kodierende Nukleotidsequenzen
überexprimiert.

12. Rekombinante Mikroorganismen aus der Familie Enterobacteriaceae, die L-Lysin produzieren und wobei man mindestens:
a) das für die Phosphoenolpyruvat-Carboxykinase kodierende pckA-Gen ausschaltet und
b) das rseB-Gen, das für den Regulator der SigmaE-Faktor-Aktivität kodiert, oder für das rseB-Genprodukt kodierende Nukleotidsequenzen überexprimiert.

13. Mikroorganismen nach Anspruch 11 bis 12, wobei die Mikroorganismen aus der Gattung Escherichia stammen.

14. Mikroorganismen nach Anspruch 13, wobei die Mikroorganismen aus der Spezies E. coli stammen.

## Revendications

1. Procédé de production d'acides L-aminés choisis dans le groupe constitué de la L-thréonine et de la L-lysine, ou d'additifs pour aliments pour animaux comprenant ces composés, par fermentation de microorganismes de la famille des Enterobacteriaceae dans lesquels le gène rseB qui code pour le régulateur de l'activité du facteur sigmaE ou des séquences nucléotidiques qui codent pour le produit du gène rseB est ou sont surexprimé(es), et isolement dudit acide L-aminé.

2. Procédé selon la revendication 1, **caractérisé en ce que** l'on emploie des microorganismes recombinants qui sont générés par transformation, transduction ou conjugaison d'un microorganisme de la famille des Enterobacteriaceae avec un vecteur, le vecteur contenant un gène rseB.

3. Procédé selon la revendication 1, **caractérisé en ce que** le nombre de copies du gène rseB chez les microorganismes recombinants augmente d'au moins une copie.

4. Procédé selon la revendication 3, **caractérisé en ce que** l'augmentation du nombre de copies du gène rseB d'au moins une copie chez les microorganismes recombinants est obtenue par l'intégration du gène dans le chromosome du microorganisme.

5. Procédé selon la revendication 3, **caractérisé en ce que** l'augmentation du nombre de copies du gène rseB d'au moins une copie chez les microorganismes recombinants est obtenue par l'utilisation d'un vecteur à réplication extrachromosomique.

6. Procédé selon la revendication 1, **caractérisé en ce que** l'on utilise le gène rseB placé sous le contrôle d'un promoteur.

7. Procédé selon la revendication 1, **caractérisé en ce que** l'on emploie des microorganismes choisis parmi les genres Escherichia, Erwinia, Providencia et Serratia.

8. Procédé selon la revendication 1, **caractérisé en ce que**, pour la production de L-thréonine, des microorganismes de la famille des Enterobacteriaceae dans lesquels, en outre, en même temps, un ou plusieurs des gènes choisis dans le groupe constitué par :
8.1 l'opéron thrABC qui code pour l'aspartate kinase, l'homosérine déshydrogénase, l'homosérine kinase et la thréonine synthase,
8.2 le gène pyc qui code pour la pyruvate carboxylase,
8.3 le gène pps qui code pour la phosphoénolpyruvate synthase,
8.4 le gène ppc qui code pour la phosphoénolpyruvate carboxylase,
8.5 les gènes pntA et pntB qui codent pour la transhydrogénase,
8.6 le gène rhtB d'Escherichia coli qui code pour une protéine qui confère une résistance à l'homosérine,
8.7 le gène mqo qui code pour la malate:quinone oxydoréductase,
8.8 le gène rhtC d'Escherichia coli qui code pour une protéine qui confère une résistance à la thréonine,
8.9 le gène thrE qui code pour la protéine d'exportation de la thréonine,
8.10 le gène gdhA qui code pour la glutamate déshydrogénase,
8.11 le gène hns qui code pour la protéine HLP-II se liant à l'ADN,
8.12 le gène pgm qui code pour la phosphoglucomutase,
8.13 le gène fba qui code pour la fructose bisphosphate aldolase,
8.14 le gène ptsH qui code pour la phosphohistidine protéine hexose phosphotransférase,
8.15 le gène ptsI qui code pour l'enzyme I du système phosphotransférase,
8.16 le gène crr qui code pour le composant IIA spécifique du glucose,
8.17 le gène ptsG qui code pour le composant IIBC spécifique du glucose,
8.18 le gène lrp qui code pour le régulateur du régulon de la leucine,
8.19 le gène csrA qui code pour le régulateur global Csr,
8.20 le gène fadR qui code pour le régulateur du régulon fad,
8.21 le gène iclR qui code pour le régulateur du métabolisme central intermédiaire,
8.22 le gène mopB qui code pour la protéine chaperonne de 10 kD,
8.23 le gène ahpC qui code pour la petite sous-unité de l'alkyl hydroperoxyde réductase,
8.24 le gène ahpF qui code pour la grande sous-unité de l'alkyl hydroperoxyde réductase,
8.25 le gène cysK qui code pour la cystéine synthase A,
8.26 le gène cysB qui code pour le régulateur du régulon cys,
8.27 le gène cysJ qui code pour la flavoprotéine de la NADPH sulfite réductase,
8.28 le gène cysI qui code pour l'hémoprotéine de la NADPH sulfite réductase,
8.29 le gène cysH qui code pour l'adénylylsulfate réductase,
8.30 le gène phoB qui code pour le régulateur positif PhoB du régulon pho,
8.31 le gène phoR qui code pour la protéine capteur du régulon pho,
8.32 le gène phoE qui code pour la protéine E de la membrane cellulaire externe,
8.33 le gène pykF qui code pour la pyruvate kinase I stimulée par le fructose,
8.34 le gène pfkB qui code pour la 6-phosphofructokinase II,
8.35 le gène malE qui code pour la protéine de liaison périplasmique du transport du maltose,
8.36 le gène sodA qui code pour la superoxyde dismutase,
8.37 le gène rseA qui code pour une protéine membranaire présentant une activité anti-sigmaE,
8.38 le gène rseC qui code pour un régulateur global du facteur sigmaE,
8.39 le gène sucA qui code pour la sous-unité décarboxylase de la 2-cétoglutarate déshydrogénase,
8.40 le gène sucB qui code pour la sous-unité dihydrolipoyltranssuccinase E2 de la 2-cétoglutarate déshydrogénase,
8.41 le gène sucC qui code pour la sous-unité β de la succinyl-CoA synthétase,
8.42 le gène sucD qui code pour la sous-unité α de la succinyl-CoA synthétase,
8.43 le gène adk qui code pour l'adénylate kinase,
8.44 le gène hdeA qui code pour une protéine périplasmique ayant une fonction de type chaperonine,
8.45 le gène hdeB qui code pour une protéine périplasmique ayant une fonction de type chaperonine,
8.46 le gène icd qui code pour l'isocitrate déshydrogénase,
8.47 le gène mglB qui code pour la protéine de transport périplasmique de liaison au galactose,
8.48 le gène lpd qui code pour la dihydrolipoamide déshydrogénase,
8.49 le gène aceE qui code pour la composante E1 du complexe pyruvate déshydrogénase,
8.50 le gène aceF qui code pour la composante E2 du complexe pyruvate déshydrogénase,
8.51 le gène pepB qui code pour l'aminopeptidase B et
8.52 le gène aldH qui code pour l'aldéhyde déshydrogénase,
est ou sont surexprimés, sont fermentés.

9. Procédé selon la revendication 1 ou 8, **caractérisé en ce que**, pour la production de L-thréonine, des microorganismes de la famille des Enterobacteriaceae dans lesquels, en outre, en même temps, un ou plusieurs des gènes choisis dans le groupe constitué par :
9.1 le gène tdh qui code pour la thréonine déshydrogénase,
9.2 le gène mdh qui code pour la malate déshydrogénase,
9.3 le produit génique du cadre ouvert de lecture (orf) yjfA d'E. coli, lorsque ledit microorganisme est Escherichia coli,
9.4 le produit génique du cadre ouvert de lecture (orf) ytfP d'E. coli, lorsque ledit microorganisme est Escherichia coli,
9.5 le gène pckA qui code pour la phosphoénolpyruvate carboxykinase,
9.6 le gène poxB qui code pour la pyruvate oxydase,
9.7 le gène aceA qui code pour l'isocitrate lyase,
9.8 le gène dgsA qui code pour le régulateur DgsA du système phosphotransférase,
9.9 le gène fruR qui code pour le répresseur du fructose,
9.10 le gène rpoS qui code pour le facteur sigma³⁸,
9.11 le gène aspA qui code pour l'aspartate ammonium lyase, et
9.12 le gène aceB qui code pour la malate synthase A,
est ou sont éliminés, sont fermentés.

10. Procédé selon l'une ou plusieurs des revendications 1 à 9, dans lequel
a) l'acide L-aminé souhaité est concentré dans le bouillon de fermentation ou dans les cellules des microorganismes, et
b) le ou les produits contenant l'acide L-aminé souhaité est ou sont isolés, la biomasse et/ou d'autres constituants du bouillon de fermentation restant éventuellement dans le produit en une quantité comprise entre > 0 et 100 %.

11. Microorganismes recombinants de la famille des Enterobacteriaceae qui produisent de la L-thréonine et dans lesquels au moins :
a) l'opéron thrABC dont les gènes codent pour l'aspartate kinase, l'homosérine déshydrogénase, l'homosérine kinase et la thréonine synthase, et
b) le gène rseB, codant pour le régulateur de l'activité du facteur sigmaE, ou des séquences nucléotidiques qui codent pour le produit du gène rseB,
sont surexprimés.

12. Microorganismes recombinants de la famille des Enterobacteriaceae qui produisent de la L-lysine et dans lesquels au moins :
a) le gène pckA codant pour la phosphoénolpyruvate carboxykinase est éliminé, et
b) le gène rseB, codant pour le régulateur de l'activité du facteur sigmaE, ou des séquences nucléotidiques qui codent pour le produit du gène rseB est ou sont surexprimé(es).

13. Microorganismes selon la revendication 11 ou 12, les microorganismes provenant du genre Escherichia.

14. Microorganismes selon la revendication 13, les microorganismes provenant de l'espèce E. coli.
